(19) Europäisches Patentamt
European Patent Office
Office européen des brevets

(11) EP 1 767 243 A1

(12) EUROPEAN PATENT APPLICATION
published in accordance with Art. 158(3) EPC

(43) Date of publication:
28.03.2007 Bulletin 2007/13

(21) Application number: 05745927.3

(22) Date of filing: 25.05.2005

(51) Int Cl.:
*A61N 1/30* (2006.01) *C08J 5/22* (2006.01)

(86) International application number:
PCT/JP2005/010009

(87) International publication number:
WO 2005/115534 (08.12.2005 Gazette 2005/49)

(84) Designated Contracting States:
DE GB

(30) Priority: 26.05.2004 JP 2004156594

(71) Applicant: TOKUYAMA CORPORATION
Shunan-shi, Yamaguchi-ken 745-8648 (JP)

(72) Inventors:
- NAGASHIMA, Masumi
Shunan-shi
Yamaguchi 7458648 (JP)
- FUKUTA, Kenji
Shunan-shi
Yamaguchi 7458648 (JP)
- SAKATA, Kanji
Shunan-shi
Yamaguchi 7458648 (JP)

(74) Representative: Cresswell, Thomas Anthony
J.A. KEMP & CO.
14 South Square
Gray's Inn
London WC1R 5JJ (GB)

# (54) IONTOPHORESIS APPARATUS

(57) An comprising (A) a working electrode structure being equipped with a working electrode, an ion-exchange membrane and a medicine-containing portion which contains an ionic medicine, (B) a counter electrode structure being equipped with an electrode opposing said working electrode and (C) a power source unit electrically connected to the working electrode structure and to the counter electrode structure, said ionic medicine being permeated into a living body by the electrophoresis through the ion-exchange membrane;
wherein said ion-exchange membrane has, as an ion-exchange resin, a crosslinked (meth)acrylic resin having a (meth)acrylic structural unit A to which an ion-exchange group is bonded. The iontophoresis device using the above ion-exchange membrane is capable of efficiently administering ionic medicines having ionic formula weights of not smaller than 500 into the living body.

Fig. 1

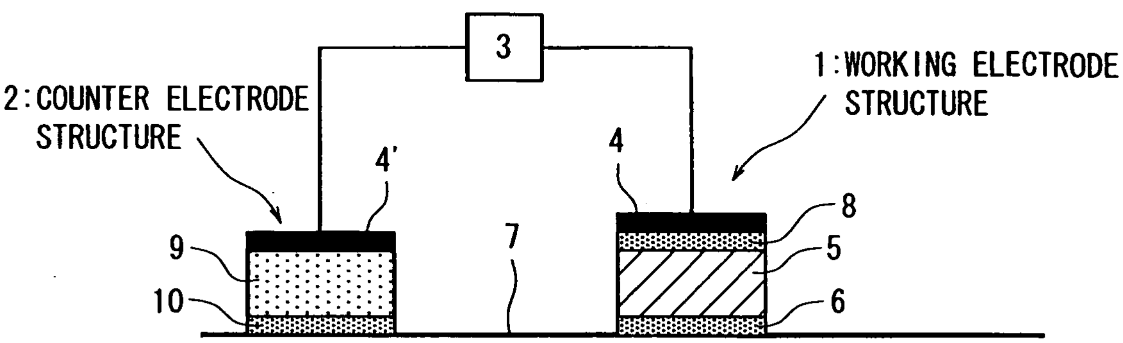




Printed by Jouve, 75001 PARIS (FR)

## Description

(Field of the Invention)

The present invention relates to an iontophoresis device for carrying out the iontophoresis for permeating, into the living body, an ionic medicine useful for the living body by utilizing the electrophoresis. More specifically, the invention relates to an iontophoresis device which uses an ion-exchange membrane and to an ion-exchange membrane used for the above device.

(Background Art)

The iontophoresis for permeating, into the living body, an ionic medicine useful for the living body by utilizing the electrophoresis has been widely known as a method of administering a medicine of a required amount into a diseased part in a pain-free state.

In the iontophoresis, so far, a medicine-containing layer imbibing an ionic medicine is placed on the living body, an working electrode is arranged on the side opposite to the living body with the medicine layer sandwiched therebetween, a counter electrode is placed on the living body separated away from the medicine-containing layer, and an electric current is permitted to flow across the working electrode and the counter electrode from a power source causing medicinal ions of the ionic medicine to permeate into the living body. This method has an object of permeating the ionic medicine only into the living body through the living body interface such as the skin and the mucous membrane. According to this method, however, the ionic medicine does not necessarily pass through the living body interface but, conversely, it often happens that sodium cations, potassium cations and chloride anions permeate back into the medicine layer from the side of the living body. In particular, ionic medicines (medicinal ions) that are considered to be useful for the living body have a smaller mobility than those of ions existing in the living body, and a desired medicine is not efficiently administered (does not efficiently permeate into the living body) in proportion to the time the electric current is supplied. In the iontophoresis, further, the medicine comes into direct contact with the electrodes triggering a reaction on the electrodes not only wasting the medicine but also forming compounds that may adversely affect the living body. Moreover, the medicine is usually used in the form of an aqueous solution. Therefore, the electrolysis of water takes place on the working electrode and on the counter electrode, whereby the pH of the medicine-containing aqueous solution varies due to $H^+$ ions and $OH^-$ ions that are formed often causing the living body to be inflamed.

In order to solve these problems, new iontophoretic methods have been proposed by arranging an ion-exchange membrane on the living body interface so that medicinal ions permeate into the living body through the ion-exchange membrane (e.g., see patent documents 1 to 4).

[Patent document 1] JP-A-3-94771
[Patent document 2] JP-T-3-504343
[Patent document 3] JP-A-4-297277
[Patent document 4] JP-A-2000-229128

According to the systems proposed in the above patent documents, the ion-exchange membrane arranged on the living body interface permits the permeation of only those ions having the same polarity as the desired medicinal ions. This makes it possible to prevent the ions having polarity opposite to that of medicinal ions of the desired medicine from oozing out of the living body and, hence, to accomplish a high dosage of the medicine as compared with when no ion-exchange membrane is arranged. The above technologies use a commercial ion-exchange membrane which employs, as a reinforcing member (base member), a woven fabric placed in the market, that is used for the manufacture of the salt and for the dialysis of food compounds. As the ion-exchange membrane, there has been used the one employing a styrene/divinylbenzene copolymer as a base resin of ion-exchange resin as a base resin, and introducing ion-exchange groups such as sulphonic acid groups and ammonium salt groups into the base resin (hereinafter called styrene-type ion-exchange membrane).

According to the study conducted by the present inventors, however, when the styrene-type ion-exchange membrane is used, the ionic medicine can be favorably administered if it has a relatively small ionic formula weight like ascorbic acid (salt) or histamine (salt). It was, however, learned that the administering efficiency sharply decreases as the medicinal ions of the ionic medicine increase, and becomes very poor as the ionic formula weight exceeds about 500.

(Summary of the Invention)

It is, therefore, an object of the present invention to provide an iontophoresis device which is capable of efficiently administering, into the living body, not only ionic medicines having small formula weights but also ionic medicines of which the medicinal ions have large formula weights.

Another object of the present invention is to provide an ion-exchange membrane used for the above iontophoresis device and a method of its production.

In an attempt to solve the above problems, the present inventors have conducted the study extensively. As a result, the inventors have discovered the fact that medicinal ions of an ionic medicine having a large ionic formula weight can be efficiently administered when there is used an ion-exchange membrane that has, as an ion-exchange resin, a crosslinked (meth)acrylic acid resin having an ion-exchange group, and have finished the present invention.

According to the present invention, there is provided an iontophoresis device comprising (A) a working electrode structure being equipped with a working electrode, an ion-exchange membrane and a medicine-containing portion which contains an ionic medicine, (B) a counter electrode structure being equipped with an electrode opposing said working electrode and (C) a power source unit electrically connected to the working electrode structure and to the counter electrode structure, said ionic medicine being permeated into a living body by electrophoresis through the ion-exchange membrane;

wherein said ion-exchange membrane has, as an ion-exchange resin, a crosslinked (meth)acrylic resin having a (meth) acrylic structural unit A to which an ion-exchange group is bonded.

The present invention further provides an ion-exchange membrane for iontophoresis having, as an ion-exchange resin, a crosslinked (meth)acrylic resin that has a (meth)acrylic structural unit A to which an ion-exchange group is bonded.

According to the present invention, there is further provided a method of producing an ion-exchange membrane for iontophoresis comprising steps of:

> contacting to a porous base member, a polymerizable solution that contains a crosslinking agent, a polymerization initiator and a polymerizable monomer composition containing a (meth)acrylic acid derivative that has an ion-exchange group, so as to permeate the polymerizable solution into voids in the porous member; and
> polymerizing the polymerizable solution.

The iontophoresis device of the present invention is capable of efficiently administering, into the living body, not only ionic medicines having small formula weights but also medicinal ions of formula weights of about 300 to about 1,000 which could be administered very poorly efficiently when a styrene-type ion-exchange membrane was used. Besides, use of the ion-exchange membrane does not almost permit ions to permeate back to the side of the medicine layer from the side of the living body. Therefore, the device works very excellently for percutaneously administering medicinal ions of relatively high molecular weights.

(Brief Description of the Drawings)

> Fig. 1 is a view schematically illustrating a representative structure of an iontophoresis device of the present invention;
> Fig. 2 is a view schematically illustrating a device used for measuring the amounts of administering the medicine according to the embodiment; and
> Fig. 3 is a sectional view schematically illustrating a portable iontophoresis device in which all constituent parts are incorporated in an armoring material.

(Best Mode for Carrying Out the Invention)

The iontophoresis device of the present invention is used for administering an ionic medicine into a living body through an ion-exchange membrane by utilizing the electrophoresis, and is constituted, as shown in Fig. 1, by a working electrode structure 1, a counter electrode structure 2, and a power source unit 3 electrically connected to these structures.

(Working electrode structure 1)

The working electrode structure 1 includes an electrode (working electrode) 4 that serves as a working electrode, a medicine-containing portion 5 containing an ionic medicine, and an ion-exchange membrane 6. The ion-exchange membrane 6 selectively permits the permeation of ions of the same polarity as the medicinal ions of the ionic medicine to be administered. In the working electrode structure 1 as shown in Fig. 1, there are arranged the working electrode 4, medicine-containing portion 5 and ion-exchange membrane 6 in this order. Usually, these members are laminated in an armoring material (not shown) to constitute the working electrode structure 1, and the ion-exchange membrane 6 is arranged to be positioned on a living body interface (skin) 7.

An ion-exchange membrane 8 may further be included between the electrode and the medicine-containing layer to prevent the decomposition of the medicine to be administered and to prevent the pH of the medicine-containing portion 5 from being varied by the electrode reaction. It is desired that the ion-exchange membrane 8 is the one which

selectively permits the passage of ions of a polarity opposite to that of the medicinal ions.

As required, further, an ion-permeating sheet made of an ionically conducting gel, a porous film or a woven fabric may be provided between the ion-exchange membrane 6 and the living body interface 7. The gel or the sheet may assume a structure integral with the working electrode structure 1. Or, the gel or the sheet may be held relative to the living body interface 7 only at the time of use. Though not illustrated, the working electrode structure 1 may further include an ionically conducting gel, an ionically electrolytic solution, or a porous film or a woven fabric imbibing the ionically electrolytic solution between the working electrode 4 and the ion-exchange membrane 8.

As the working electrode 4 in the working electrode structure 1, there can be used, without limitation, any electrode that is usually used in the electrochemical processes. For example, there can be used an electrode comprising an electronically conducting material such as gold, platinum, silver, copper, nickel, zinc or carbon, or a self-sacrificing electrode such as semiconductor electrode or silver/silver chloride, which may be used alone or in combination. Preferably, there can be exemplified gold, platinum, silver and carbon. These electrodes may be an amorphous laminate of plates, sheets, meshes or fibers, which is shaped and worked like a paper, or may be the one obtained by plating or vaporizing an electrode member on an ion-exchange membrane.

As the medicine-containing portion in the working electrode structure 1, there can be used, without any limitation, a medicine-containing layer that is used in the ordinary iontophoresis. That is, there can be used a solution obtained by dissolving an ionic medicine in a solvent such as water or ethanol, a gel obtained by mixing the above solution with a polyvinyl alcohol or a polyvinyl pyrrolidone, or the one of a porous film or a gauze imbibing the above solution. There is no particular limitation on the ionic medicine contained in the medicine-containing portion 5. The ionic medicine may be any substance that comprises cations and anions and exhibits medicinal effect as the positive ions or negative ions enter into the living body.

Examples of the ionic medicine of which the positive ions exhibit the effect include anesthetics such as procaine hydrochloride, lidocaine hydrochloride and dibucaine hydrochloride; anti-malignant tumor agents such as mitomycin and pleomycin hydrochloride; anodynes such as morphine hydrochloride; steroids such as medroxyprogesterone acetate; histamine and insulin. As the ionic medicine of which the negative ions exhibit the effect, there can be exemplified vitamin agents such as vitamin B2, vitamin B12, vitamin C, vitamin E and folic acid; anti-inflammatory agents such as aspirin and ibuprofen; adrenocortical hormones such as dexamethasone-type water-soluble compounds; and antibiotics such as benzylpenicillin potassium. Among them, the ionic medicine of which the medicinal ions have a formula weight of 300 to 1500 and, particularly, 400 to 1,000 can be administered at a higher efficiency by using the iontophoresis device of the present invention than by using the conventional devices.

The ion-exchange membrane 6 has a structure in which, for example, voids in the porous base member are filled with an ion-exchange resin. According to the present invention, it is very important that the ion-exchange resin in the ion-exchange membrane 6 is a crosslinked (meth)acrylic resin having a (meth)acrylic structural unit A to which an ion-exchange group is bonded. Use of the above ion-exchange membrane 6 makes it possible to efficiently administer even medicinal ions having a large ionic formula weight into the living body. When, for example, an ion-exchange membrane having a styrene-type ion-exchange resin is used, it is not allowed to efficiently administer the medicinal ions having a large ionic formula weight. Further, a (meth)acrylic resin without the crosslinking structure, though it may have the above (meth)acrylic structural unit A (ion-exchanging structural unit), dissolves in a solvent such as water that is used for dissolving the ionic medicine, and is not capable of exhibiting the function of the ion-exchange membrane in the iontophoresis device. Moreover, though the reason is not clear, the ion-exchange membrane comprising an uncrosslinked resin permits the medicine to permeate through less.

Use of the ion-exchange membrane 6 having, as an ion-exchange resin, a crosslinked (meth)acrylic resin that has a (meth)acrylic structural unit A to which an ion-exchange group is bonded which is contemplated by the present invention, makes it possible to efficiently and selectively administer the medicinal ions having a large ionic formula weight though the reason has not been clarified yet. The inventors, however, presume it as described below. That is, the above-mentioned ion-exchange crosslinked (meth)acrylic resin has a soft structure such as an ester structure or an amide structure between a polymer chain and an ion-exchange group enabling the ion-exchange group to move relatively freely in the ion-exchange membrane (resin). Therefore, when the medicinal ions having a large ionic formula weight permeate through, it is presumed that the ion-exchange groups flexibly move to little interrupt the migration of medicinal ions. That is, the ion-exchange membrane having a styrene-type ion-exchange resin is more rigid than the (meth)acrylic resin providing a low degree of freedom for the ion-exchange groups and making it difficult to permeate medicinal ions having a large ionic formula weight. Besides, the uncrosslinked (meth)acrylic resin permits the film thereof to dissolve in a solvent and permits the medicine to permeate through less.

The above ion-exchange crosslinked (meth)acrylic resin (hereinafter called crosslinked (meth)acrylic ion-exchange resin) used in the present invention has a crosslinked structure offering advantages of excellent strength and easy formation into a film.

The crosslinked (meth)acrylic ion-exchange resin has a (meth)acrylic structural unit A (ion-exchange structural unit A) to which an ion-exchange group is bonded in a (meth)acrylic polymer chain. The ion-exchange structural unit A

is typically represented by the following formula (1):

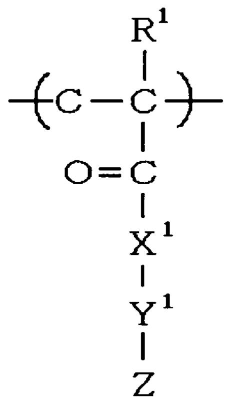

(1)

wherein,

$R^1$ is a hydrogen atom or a methyl group,
$X^1$ is -O- or >NR' (where R' is a hydrogen atom or a monovalent organic group without ion-exchange group),
$Y^1$ is a bonding hand or a divalent organic group, and
Z is an ion-exchange group.

In the above formula (1), R' in >NR' (group $X^1$) is a hydrogen atom or a monovalent organic group. There is no particular limitation on the monovalent organic group provided it has no ion-exchange group. There can be exemplified a monovalent organic group having, preferably, 1 to 20 carbon atoms and, more preferably, 1 to 10 carbon atoms, e.g., alkyl groups such as methyl group, ethyl group, propyl group, butyl group and hexyl group; hydroxyl group-substituted alkyl groups such as 2-hydroxyethyl group; and halogen atom-substituted alkyl groups such as 2-chloroethyl group and the like.

Concerning the bonding hand or the divalent organic group denoted by $Y^1$, there is no particular limitation on the divalent organic group. There can be exemplified divalent organic groups having, preferably, 1 to 30 carbon atoms and, more preferably, 2 to 20 carbon atoms, e.g., alkylene groups such as methylene group, ethylene group, propylene group, trimethylene group, 2-methylpropylene group, hexamethylene group and decamethylene group; hydroxyl group-substituted alkylene groups such as 2- or 3-hydroxytrimethylene group; halogen atom-substituted alkylene groups such as 2-trichlorotrimethylene group; divalent groups derived from an alkyleneoxy group represented by the following formula:

$$-(CH_2CH_2O)_n-CH_2CH_2-$$

or

$$-(CH_2CH(CH_3)O)_m-CH_2CH(CH_3)-$$

(wherein n is an integer of 1 to 9, and m is an integer of 1 to 6); and a group represented by the following formula:

$$-CH_2CH_2-OC(O)CH_2CH_2-,$$

or

$$-CH_2CH_2-(OC(O)CH_2CH_2CH_2CH_2CH_2)_1-$$

(wherein 1 is 1 or 2).

There is no particular limitation on the ion-exchange group (group Z) in the above formula (1) provided it is a functional group capable of becoming a negative or positive electric charge in an aqueous solution. Concrete examples of the ion-exchange group include carboxylic acid group (-COOH), phosphoric acid group {$-O-P(O)(OH)_2$}, phosphonic acid group {$-P(O)(OH)_2$}, and metal salt groups and onium salt groups corresponding to these acid groups, which are the cation-exchange groups, as well as primary to tertiary amino groups, quaternary ammonium group, pyridyl group, imidazole group, quaternary pyridinium group and quaternary imidazolinium group, which are the anion-exchange groups.

Further, the crosslinked (meth)acrylic ion-exchange resin used in the present invention may have another structural unit B represented by the following formula (2):

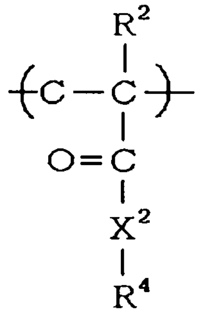

(2)

wherein,

$R^2$ is a hydrogen atom or a methyl group,
$X^2$ is -O- or >NR' (where R' is as defined above), and
$R^4$ is a monovalent organic group without ion-exchange group,

as a structural unit for constituting the (meth)acrylic polymer chain, in addition to the ion-exchange structural unit A represented by the above formula (1).

In the above formula (2), R' in >NR' (divalent group $X^2$) is a hydrogen atom or a monovalent organic group. There is no particular limitation on the monovalent organic group like that of the above formula (1), and concrete examples of the above organic group will be the same as those exemplified above concerning the formula (1).

There is no particular limitation on the group $R^4$ provided it is a monovalent organic group without ion-exchange group. Usually, however, it is desired that the group $R^4$ is an organic group having 1 to 30 carbon atoms and, particularly, 1 to 20 carbon atoms. Concrete examples of the organic group include alkyl groups such as methyl group, ethyl group, propyl group, butyl group, hexyl group, 2-ethylhexyl group, isodecyl group, n-lauryl group, cyclohexyl group and isobornyl group; hydroxyl group-substituted alkyl groups such as 2-hydroxyethyl group, 2- or 3-hydroxypropyl group and 2-hydroxybutyl group; halogen atom-substituted alkyl groups such as 2-chloroethyl group; a chain ether group with its terminal capped with an alkyl group represented by the following formula:

$$-(CH_2CH_2O)_n-CH_3$$

or

$$-(CH_2CH(CH_3)O)_m-CH_3$$

(wherein n is an integer of 1 to 9, and m is an inter of 1 to 6), a chain ether group having a hydroxyl group at its terminal represented by,

$$-(CH_2CH_2O)_n-H$$

$$-(CH_2CH(CH_3)O)_m-H$$

(wherein n is an integer of 1 to 9, and m is an integer of 1 to 6),
and groups having a cyclic ether structure, such as tetrahydrofurfuryl group and glycidyl group.

Upon having a crosslinked structure, the crosslinked (meth)acrylic ion-exchange resin used in the present invention has a crosslinked structural unit which has a structural unit C (crosslinked structural unit) represented by the following formula (3):

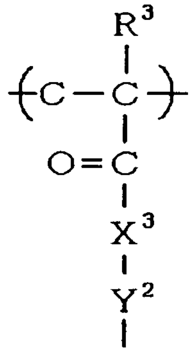

(3)

wherein,

$R^3$ is a hydrogen atom or a methyl group,
$X^3$ is O or NR' (where R' is as defined above), and
$Y^2$ is a divalent organic group forming a crosslinked chain.

In the above formula (3), R' in >NR' (divalent group $X^2$) is a hydrogen atom or a monovalent organic group without ion-exchange group like that of the formula (1) above. Concrete examples of the above organic group are the same as those exemplified above concerning the formula (1). Further, the divalent organic group $Y^2$ forming the crosslinked chain is the same as the one exemplified as the group $Y^1$ in the above formula (1).

In the crosslinked (meth)acrylic ion-exchange resin having the above structural units A to C, the structural units may be arranged in any order, the structural units may have their own structures, or the structural units may, further, have a plurality of structural units, respectively. Further, when the sum of these structural units A to C is regarded to be 1, it is desired that the structural unit A (ion-exchange structural unit) is contained at a ratio of 0.05 to 0.9995, particularly, 0.10 to 0.999 and, most desirably, 0.30 to 0.99, the structural unit B is contained at a ratio of 0 to 0.9495, particularly, 0 to 0.80 and, most desirably, 0 to 0.60, and the structural unit C (crosslinked structural unit) is contained at a ratio of 0.0005 to 0.950, particularly, 0.001 to 0.50 and, most desirably, 0.01 to 0.30.

The crosslinked (meth)acrylic ion-exchange resin used in the present invention may, as required, contain structural units (structural units based on a polymerizable monomer other than the (meth)acrylic ones), such as a vinyl structural unit other than the (meth)acrylic one. Here, however, the ratio of such other structural units is not larger than 1, preferably, not larger than 0.5 and, more preferably, not larger than 0.1 with respect to 1 which is the sum of the above structural units A, B and C.

Though there is no particular limitation on the crosslinked (meth)acrylic ion-exchange resin having the above-mentioned various kinds of structural units, the crosslinked (meth)acrylic ion-exchange resin is obtained by polymerizing a mixture of monomers containing polymerizable monomers of the type of (meth)acrylic acid derivatives corresponding to the above structural units A, B and C in amounts that satisfy the above-mentioned ratios of amounts.

As the (meth)acrylic acid derivative-type polymerizable monomers corresponding to the structural units A and B, for example, there can be exemplified the following ones.

- Polymerizable monomers corresponding to the structural unit A (ion-exchange structural unit) -

(a) Monomers having a cation-exchange group.

Sulfonic acid-type monomers such as 2-(meth)acrylamide-2-methylpropanesulfonic acid, 3-sulfopropane (meth) acrylate, 10-sulfodecane (meth)acrylate and salts thereof;
Carboxylic acid-type monomers such as 2-(meth)acryloylethylphthalic acid, 2-(meth)acryloylethylsuccinic acid, 2-(meth)acryloylethylmaleic acid, 2-(meth)acryloylethyl-2-hydroxyethylphthalic acid, 11-(meth)acryloyloxydecyl-1,1-dicarboxylic acid, 4-(meth)acryloyloxyethyltrimeritic acid and salts thereof; and
Phosphoric acid-type monomers such as 2-(meth)acryloyloxyethyl dihydrogenphosphate, 2-(meth)acryloyloxyethyl phenyl hydrogenphosphate, 10-(meth)acryloyloxydecyl dihydrogenphosphate, 6-(meth)acryloyloxyhexyl dihydrogenphosphate, 2-(meth)acryloyloxyethyl 2-bromoethyl hydrogenphosphate and salts thereof.

(b) Monomers having an anion-exchange group.

N,N-dimethylaminoethyl(meth)acrylate,
N,N-diethylaminoethyl(meth)acrylate,
N,N-dimethylaminoethyl(meth)acrylate/methyl chloride, and
N,N-diethylaminoethyl(meth)acrylate/methyl chloride.

- Polymerizable monomers corresponding to the structural unit B -

(Meth)acrylates such as:

Methy(meth)acrylate,
Ethyl(meth)acrylate,
Isopropyl(meth)acrylate,
Tetrahydrofurfuryl(meth)acrylate,
Glycidyl(meth)acrylate,
2-Hydroxyethyl(meth)acrylate,
2-Hydroxypropyl(meth)acrylate,
3-Hydroxypropyl(meth)acrylate,
2,3-Dihydroxybutyl(meth)acrylate,
2,4-Dihydroxybutyl(meth)acrylate,
2-Hydroxymethyl-3-hydroxypropyl(meth)acrylate,
2,3,4-Trihydroxybutyl(meth)acrylate,
2,2-Bis(hydroxymethyl)-3-hydroxypropyl(meth)acrylate,
2,3,4,5-Tetrahydroxypentyl(meth)acrylate,
Diethylene glycol mono(meth)acrylate,
Triethylene glycol mono(meth)acrylate,
Tetraethylene glycol mono(meth)acrylate, and
Pentaethylene glycol mono(meth)acrylate.

The polymerizable monomer corresponding to the structural unit C (crosslinked structural unit) is a monomer in which one or a plurality of polymerizable groups copolymerizable with (meth)acrylic groups are bonded to the group $Y^3$ (crosslinked chain) in the structural unit in the above formula (3), and is a polyfunctional (meth)acrylic acid derivative-type polymerizable monomer that works as a crosslinking agent. There can be exemplified the following monomers.

- Polymerizable monomers corresponding to the structural unit C (crosslinked structural unit) -

Ethylene glycol di(meth)acrylate,
Diethylene glycol di(meth)acrylate,
Triethylene glycol di(meth)acrylate,
Tetraethylene glycol di(meth)acrylate,
Nonaethylene glycol di(meth)acrylate,
Tetradecaethylene glycol di(meth)acrylate,
Butylene glycol di(meth)acrylate,
Neopentyl glycol di(meth)acrylate,
Propylene glycol di(meth)acrylate,
1,3-Butanediol di(meth)acrylate,
1,4-Butanediol di(meth)acrylate,
1,6-Hexanediol di(meth)acrylate,
1,9-Nonanediol di(meth)acrylate,
1,10-Decanediol di(meth)acrylate,
Glycerine di(meth)acrylate,
Trimethylolpropane tri(meth)acrylate,
Methylene bis(meth)acrylamide, and

Hexamethylene di(meth)acrylamide.

The (meth)acrylic acid derivative-type polymerizable monomers corresponding to the above structural units A, B and C may be used in a single kind or may be used in a combination of two or more kinds. They are used in such amounts that the ratio of amounts of the structural units A to C lie in the above-mentioned ranges. As required, further, any other polymerizable monomers (e.g., N-vinylpyrrolidone, vinyl acetate and methyl vinyl ketone) corresponding to vinyl-type structural units other than those of the (meth)acrylic type may be copolymerized in combination.

Though there is no particular limitation on the method of polymerizing the polymerizable monomers, a solution of a mixture of polymerizable monomers is, usually, blended with a thermal polymerization initiator and is heated. As the thermal polymerization initiator, there can be particularly preferably used organic peroxides such as benzoyl peroxide, t-butylperoxy-2-ethyl hexanoate, t-butylperoxy dicarbonate, diisopropylperoxy dicarbonate, dilauroylperoxide, t-butylhydroperoxide, cumenehydroperoxide, di-t-butyl peroxide, dicumyl peroxide and diacetyl peroxide. The amount of the polymerization initiator is desirably about 0.1 to 20 parts by mass and, particularly, about 0.5 to about 10 parts by mass per a total of 100 parts by mass of the above polymerizable monomers.

In order to improve the miscibility of the polymerizable monomers, further, a solvent such as water or alcohol may be added to the solution of the above mixture of monomers, and a plasticizer may be blended, such as dibutyl phthalate, dioctyl phthalate, dimethyl isophthalate, dibutyl adipate, triethyl citrate, acetyltributyl citrate, dibutyl cebacate or dibenzyl ether.

To produce the ion-exchange membrane 6 by using the crosslinked (meth)acrylic ion-exchange resin obtained by polymerizing the above-mentioned polymerizable monomer according to the present invention, a polymerizable solution containing a mixture of polymerizable monomers and a thermal polymerization initiator is brought into contact with a porous base member that serves as a reinforcing member or a support member, and the polymerization is conducted in a state where the polymerizable solution is filled in voids of the porous base member. Use of the above porous base member makes it very easy to accomplish both a high strength for preventing breakage during the storage and use and flexibility for favorably following up the shape of skin at the time of use.

There is no particular limitation on the porous base member. Usually, a paper, a woven fabric, a nonwoven fabric or a porous drawn film is used. From the standpoint of obtaining the ion-exchange membrane having a small thickness and a high mechanical strength (from the standpoint of efficiently administering the medicine while effectively preventing the breakage), in particular, it is desired to use a nonwoven fabric or a porous drawn film as the porous base member and it is most desired to use a porous drawn film as the porous base member.

The porous drawn film has many fine pores penetrating through from the front surface to the back surface. To obtain both a large strength and flexibility, it is desired to use the porous film of a thermoplastic resin. As the thermoplastic resin, there can be used polyolefin resins such as homopolymers or copolymers of $\alpha$-olefins like ethylene, propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride/vinyl acetate copolymer, vinyl chloride/vinylidene chloride copolymer, and vinyl chloride/olefin copolymer; fluorine-contained resins such as polytetrafluoroethylene, polychlorotrifluoroethylene, vinylidene polyfluoride, tetrafluoroethylene/hexafluoropropylene copolymer, tetrafluoroethylene/perfluoroalkylvinyl ether copolymer and tetrafluoroethylene/ethylene copolymer; polyamides such as nylon 6 and nylon 66; and polyimide resin. It is, however, desired to use a polyolefin resin, particularly, polyethylene or polypropylene and, most desirably, polyethylene from the standpoint of mechanical strength, flexibility, chemical stability and resistance against the chemicals.

There is no particular limitation on the property of the porous drawn film comprising the above thermoplastic resin. From the standpoint of obtaining an ion-exchange membrane having a small thickness, a large strength and a low electric resistance, however, it is desired that the pores have an average diameter of, preferably, 0.005 to 5.0 $\mu$m, more preferably, 0.01 to 2.0 $\mu$m and, most preferably, 0.02 to 0.2$\mu$m. The above average porous diameter stands for a value measured in compliance with the Bubble Point Method. Similarly, it is desired that the percentage of voids is, preferably, 20 to 95% and, more preferably, 30 to 90% and, most preferably, 30 to 60%. Further, the thickness of the porous film is, preferably, 5 to 140 $\mu$m, more preferably, 10 to 120 $\mu$m and, most preferably, 15 to 55 $\mu$m so that the ion-exchange membrane will assume the thickness that will be described later. The ion-exchange membrane 6 produced by using the above porous drawn film as the porous base member has a thickness equal to about the thickness of the porous drawn film plus 0 to about 20 $\mu$m.

The porous drawn film of the above thermoplastic resin can be obtained according to the methods taught in JP-A-9-212964 and JP-A-2002-338721. Concretely speaking, the porous film is prepared by mixing an organic liquid to a thermoplastic resin to form it into a sheet or a film and, then, extracting the organic liquid therefrom by using a solvent. The porous film can be further prepared even by drawing a sheet obtained by filling the thermoplastic resin with an inorganic filler and/or an organic filler. The porous drawn film is further available in the market (for example, "Hipore" manufactured by Asahi Kasei Co., "U-Pore" manufactured by Ube Kosan Co., "Setela" manufactured by Tonen Tapils Co., "Expole" manufactured by Mitsubishi Kagaku Co., "Hilet" manufactured by Mitsui Kagaku Co., etc.).

As the nonwoven fabric used as the porous base member, there can be used those produced by a dry method

and a wet method without any particular limitation. As the material of the nonwoven fabric, there can be used, for example, polyester fiber, polypropylene fiber, polyamide fiber, nylon fiber, acrylic fiber, rayon fiber, vinylon fiber or polyurethane fiber. Though there is no particular limitation on the properties of the nonwoven fabric, it is desired that the nonwoven fabric has a weight of 20 to 100 g/m$^2$ and an apparent thickness of 30 to 250 μm from the standpoint of obtaining an ion-exchange membrane having a small thickness, excellent strength and low electric resistance. When the above nonwoven fabric is used as the base member, the ion-exchange membrane, usually, has a thickness equal to the apparent thickness of the nonwoven babric used as the base member plus 0 to minus about 30 μm.

There is no particular limitation on the method of bringing the polymerizable solution into contact with the porous base member provided the polymerizable solution permeates into the voids in the porous base member. Usually, however, an application method, a spray method or an immersion method is employed. When the polymerizable solution is to be applied or sprayed, however, a method may be employed to bring the two into contact under a reduced pressure or to apply pressure after the contact, so that the voids in the porous base member are favorably filled with the solution.

The polymerization in a state where the polymerizable solution permeates through the porous base member to fill the voids, is carried out by elevating the temperature from normal temperature while pressing the porous base member by holding it with films having smooth surfaces, such as polyester films. Upon conducting the polymerization while holding the porous base member with films, the polymerization is prevented from being adversely affected by oxygen in the environment, and the surfaces after the polymerization can be smoothed. The polymerizing conditions may be suitably determined depending upon the kind of the polymerization initiator that is used and the composition of the monomer. Usually, a state of being heated at about 80 to about 120°C is maintained for about 5 minutes to about 10 hours.

The ratio (filling ratio) of the ion-exchange resin contained in the ion-exchange film 6 obtained by the above method may vary depending upon the percentage of voids of the porous base member that is used or the amount of nonpolymerizing components in the polymerizable solution that is used but is, usually, in a range of 5 to 95% by mass and is, preferably, adjusted to lie in a range of 10 to 90% by mass and, particularly, 20 to 60% by mass in order to facilitate the permeation of medicinal ions and to increase the strength of the ion-exchange membrane.

It is, further, desired that the ion-exchange membrane 6 contains the ion-exchange groups Z in an amount of 0.1 to 8.0 mmols/g, and, particularly, 0.2 to 5.0 mmols/g as the ion-exchange capacity. As the ion-exchange capacity increases, the electric resistance of the ion-exchange membrane 6 decreases and the medicine can be administered in an increased amount at a constant voltage. If the ion-exchange capacity exceeds 5.0 mmols/g, however, the production thereof becomes difficult. If 8.0 mmols/g is exceeded, the production becomes substantially impossible.

It is further desired that the ion-exchange membrane 6 has a water content of a certain degree, such as about 5 to about 90% and, particularly, not smaller than 10% so that its electric resistance will not increase due to drying. The water content can be controlled to remain in the above range relying upon the kind of the ion-exchange groups Z introduced into the crosslinked (meth)acrylic ion-exchange resin, upon the ion-exchange capacity and upon the degree of crosslinking. In order to administer the desired medicine in large amounts, further, it is desired that the ion-exchange membrane 6 has a fixed ion concentration of 0.5 to 15.0 mmols/g - water.

It is further desired that, when a porous drawn film is used as the porous base member, the ion-exchange membrane 6 used for the iontophoresis device of the present invention has a thickness of, preferably, 5 to 150 μm, more preferably, 10 to 130 μm, and, particularly preferably, 15 to 60 μm. When a nonwoven fabric is used as the porous base member, further, the thickness of the ion-exchange membrane 6 has a thickness in a range of, desirably, 30 to 250 μm and, more desirably, 50 to 200 μm. The ion-exchange membrane 6 exhibits an increased strength when its thickness is large. The ion-exchange membrane 6, on the other hand, exhibits excellent property of following up the surface of the living body and a decreased electric resistance when its thickness is small.

When the iontophoresis device of the present invention is used in a manner that the ion-exchange membrane 6 comes into direct contact with the surface of the living body such as the skin, it is desired that the ion-exchange membrane 6 has a smooth surface from the standpoint of accomplishing intimate adhesion.

The ion-exchange membrane 6 used in the present invention may be the one produced by a method other than the above-mentioned method provided it uses, as the ion-exchange resin, a crosslinked (meth)acrylic resin having a (meth)acrylic structural unit to which an ion-exchange group is bonded. For example, the ion-exchange membrane 6 may be produced by a casting method using the above-mentioned polymerizing solution, or may be produced by forming a film of a crosslinked (meth)acrylic resin without ion-exchange group and, thereafter, introducing the ion-exchange groups into the film by a known method.

(Counter electrode structure 2)

In the iontophoresis device of the present invention, the counter electrode structure 2 has an electrode (counter electrode) 4' that opposes the working electrode 4 of the working electrode structure 1 and can assume, without any limitation, a structure used for a portion including an electrode that becomes a counter electrode in an ordinary iontophoresis device. That is, the counter electrode structure 2 may be the electrode (counter electrode 4') itself, may be a structure in which the electrode (counter electrode 4') is arranged on a sheet of an ionically conducting gel, a porous film or a woven fabric, or may be a structure in which the electrode (counter electrode 4') is arranged on an ion-exchange membrane using a porous film as the base member or on any other ion-exchange membrane. Preferably as shown in Fig. 1, the counter electrode 4', an electrolyte-containing portion 9 containing an ionic electrolyte and an ion-exchange membrane 10 are laminated in this order, the ion-exchange membrane 10 being arranged on the living body interface. In this case, the ion-exchange membrane 10 may be an ion-exchange membrane formed by using the above porous base member and the crosslinked (meth)acrylic ion-exchange resin, or may be any other ion-exchange membrane (e.g., ion-exchange membrane using a styrene-type ion-exchange resin). It is, however, desired that the ion-exchange membrane 10 is an ion-exchange membrane formed by using a porous drawn film as the porous base member from the standpoint of its excellent mechanical strength. Further, the ion-exchange membrane 10 may be the one which selectively permits the permeation of ions of a polarity same as, or opposite to, that of the medicinal ions of the desired medicine. Preferably, however, the ion-exchange membrane 10 is the one that selectively permeates ions of the polarity opposite to that of the medicinal ions of the desired medicine to prevent the permeation of the desired medicine into the counter electrode structure from the living body.

The electrolyte-containing portion 9 in the counter electrode structure 2 may be a solution itself obtained by dissolving an ionic electrolyte in a solvent such as water or an ethanol, a gel obtained by mixing the above solution with a polyvinyl alcohol or a polyvinyl pyrrolidone, or the one of a porous film or a gauze imbibing the above solution. There can be used any ionic electrolyte without limitation, such as sodium chloride or potassium chloride, if it dissolves in a solvent such as water or ethanol and exhibits ionic property.

Further, like in the case of the working electrode structure 1, the counter electrode structure 2 may be provided with an ion-exchange membrane between the counter electrode 4' and the ion-exchange membrane 10, may be provided with a sheet capable of permeating ions comprising an ionically conducting gel, a porous film or a woven fabric between the ion-exchange membrane 10 and the living body interface, or may be provided with an ionically conducting gel or an ionically electrolytic solution or with a porous film or a woven fabric imbibing the ionically electrolytic solution between the counter electrode 4' and the ion-exchange membrane closest thereto.

(Power source unit 3)

As the power source unit 3 in the iontophoresis device of the present invention, there can be used any power source unit that is used in an ordinary iontophoresis device without limitation. When the working electrode structure 1, counter electrode structure 2 and the power source unit 3 are independent from each other, there can be used an external power source that can be connected to a battery or to a power source of the system. In such a case, it is desired to use in combination a power source control system such as a system for stabilizing the voltage or the current or a system for applying a pulse current.

When the iontophoresis device of the present invention is to be realized in a portable form, it is desired to use a cell as the power source. As the cell, there can be exemplified a coin type silver oxide cell, an air-zinc cell or a lithium ion cell. By using the above small cell as a power source, there can be obtained an iontophoresis device as shown in Fig. 3, which is small in size and easy to carry incorporating the working electrode structure 1, the counter electrode structure 2 and the power source unit 3 in an armoring material 12. In fabricating the portable iontophoresis device, it is desired that the armoring material is a highly flexible resin or rubber to realize a high follow-up property to the skin shape.

There is no particular limitation on the use of the iontohoresis device of the present invention. Namely, the iontophoresis device may be used in a customary manner, usually, by bringing the working electrode structure 1 and the counter electrode structure 2 into intimate contact with the surface of the living body which is the object to where the medicine is to be permeated, and by flowing a current by applying a voltage from the power source unit 3. In this case, the ion-exchange membrane 6 in the working electrode structure 1 is so disposed as to be positioned between the medicine-containing portion 5 and the surface of the living body, so that the ions having a medicinal effect produced from the ionic medicine in the medicine-containing portion 5 permeate into the living body passing through the ion-exchange membrane 6.

(Examples)

The invention will be described more concretely by way of the following Examples and Comparative Examples to which only, however, the invention is in no way limited. Properties of the ion-exchange membranes shown in Examples and Comparative Examples were measured by the methods described below.

(1) Ion-exchange capacity, water content and fixed ion concentration:

The ion-exchange membrane was immersed in a 1 (mol/l) HCl aqueous solution for not less than 10 hours. Thereafter, in the case of the cation-exchange membrane, the hydrogen ion type was substituted by the sodium ion type with a 1 (mol/l) NaCl aqueous solution, and the liberated hydrogen ions were determined with a sodium hydroxide aqueous solution by using a potential-difference titration device (COMTITE-900, manufactured by Hiranuma Sangyo Co.)(A mols). In the case of the anion-exchange membrane, on the other hand, a chloride ion type was substituted by a nitric acid ion type with a 1 (mol/l) $NaNO_3$ aqueous solution, and the liberated chloride ions were determined with a silver nitrate aqueous solution by using the potential-difference titration device (COMTITE-900, manufactured by Hiranuma Sangyo Co.)(A mols).

Next, the same ion-exchange membrane was immersed in a 1 (mol/l) HCl aqueous solution for not less than 4 hours, and was washed with ion-exchanged water to a sufficient degree. The membrane was taken out, water on the surfaces thereof was wiped with a tissue paper or the like, and the weight (W g) thereof when wet was measured. Next, the membrane was dried at 60°C for 5 hours under a reduced pressure, and the weight was measured (D g). Based on the above measured values, the ion-exchange capacity, water content and fixed ion concentration were found in compliance with the following formulas,

$$\text{Ion-exchange capacity [mmol/g-dry weight]} = A \times 1000/D$$

$$\text{Water content [\%]} = 100 \times (W - D)/D$$

$$\text{Fixed ion concentration [mmol/g-water]} = \text{ion-exchange capacity/water content} \times 100$$

(2) Membrane resistance.

An ion-exchange membrane was held in a two-chamber cell equipped with a platinum black electrode, a 3 (mol/l) sulfuric acid aqueous solution was filled on both sides of the ion-exchange membrane, a resistance across the electrodes was measured relying on an AC bridge (frequency of 1000 cycles/sec) at 25°C, and the membrane resistance was found relying upon a difference between the resistance across the electrodes and the resistance across the electrodes of when no ion-exchange membrane was set up. The membrane used for the measurement had been equilibrated in advance in a 3 (mol/l) sulfuric acid aqueous solution.

(3) Amount of permeation of medicine through a virtual skin system.

An aqueous solution containing 10% by mass of a polyvinyl alcohol (NH-20 manufactured by Nihon Gosei Co.) was applied onto a Teflon sheet in such a manner that the thickness of the polyvinyl alcohol film after the solvent was removed was 6 μm. Thereafter, water was removed by drying conducted at 150°C for 10 minutes to obtain a virtual skin. Next, a filtering paper (filtering paper 5C for chemical analysis manufactured by Advantech Co.), the virtual skin, the ion-exchange membrane to be measured and protective ion-exchange membranes that prevent the medicine from arriving at the electrode, were set in a cell shown in Fig. 2, and the medicine solution chamber was filled with an aqueous solution of medicine of a predetermined concentration, a virtual skin chamber was filled with an aqueous solution of 0.9% by mass of sodium chloride, and the two electrode chambers were filled with a 0.1 (mol/l) sodium lactate aqueous solution.

As the protective ion exchange membrane, there was used an anion-exchange membrane obtained in Comparative Example 1 when the ion-exchange membrane to be measured was the cation-exchange membrane, and there was used a cation-exchange membrane obtained in Comparative Example 2 when the object to be measured was the anion-exchange membrane.

Next, an electric current was supplied for 3 hour at a predetermined constant current density or at a constant voltage while maintaining the cell at 25°C and stirring the medicine solution chamber and the virtual skin chamber. After the end of supplying the electric current, the solution in the virtual skin chamber was readily drained and the amount of

medicine was measured relying on a liquid chromatography. The same operation was executed without supplying the electric current to measure a blank value. A difference from the amount of medicine of when the current was supplied was calculated, and was regarded to be the amount the medicine has permeated.

(4) Amount of permeation of medicine through a living body skin system.

As living body skins, there were used a skin of a back portion of a micropig (Yucatane micropig, five months old, female) instead of using the virtual skin (cast film of polyvinyl alcohol). The amount the medicine has permeated through the living body skin system was measured by the same method as the one for the virtual skin system.

(Preparation Example 1)

A polymerizable monomer composition (polymerizable solution) was prepared by mixing the components according to the following recipe.

80% N,N-dimethylaminoethyl methacrylate/methyl chloride aqueous solution: 37 g
Nonaethyllene glycol dimethacrylate: 26 g
Hydroxyethyl methacrylate: 37 g
t-Butylperoxyethyl hexanoate: 3 g 100 Grams of the above composition was introduced into a 500-ml glass container, and in which a porous drawn film (polyethylene having a weight average molecular weight of 250,000, film thickness of 25 μm, average pore diameter of 0.03 μm, percentage of voids of 37%) measuring 12 cm x 13 cm was immersed under the atmospheric pressure at 25°C for 10 minutes, so that the polymerizable monomer composition has permeated into the voids in the porous drawn film.

Next, the porous drawn film was taken out from the glass container, and both sides of the film were covered with a polyester film of 100 μm, followed by the thermal polymerization under a nitrogen pressure of 3 $kg/cm^2$ at 70°C for 2 hours and, then, at 90°C for 3 hours to obtain a quaternary ammonium-type anion-exchange membrane.
The obtained anion-exchange membrane was measured for its ion-exchange capacity, water content, fixed ion concentration, membrane resistance and membrane thickness. The results were as shown in Table 1.

(Preparation Examples 2 to 4)

Anion-exchange membranes were prepared in the same manner as in Preparation Example 1 but changing the polymerizable monomer composition into those compositions shown in Table 1. Properties of the obtained membranes were as shown in Table 1.

(Preparation Example 5)

An anion-exchange membrane was prepared in the same manner as in Preparation Example 1 but changing the porous drawn film into a nonwoven fabric shown in Table 1. Properties of the obtained membrane were as shown in Table 1.

(Preparation Examples 6 to 8)

Cation-exchange membranes were prepared in the same manner as in Preparation Example 1 but changing the polymerizable monomer composition into those compositions shown in Table 1. Properties of the obtained membranes were as shown in Table 1.

Table 1

| Prep. Ex. | Prous base member | Composition (weight ratio) | | | | | |
|---|---|---|---|---|---|---|---|
| | | DMC | 9EG | 4EG | HEMA | P-1M | PO |
| 1 | A | 37 | 26 | | 37 | | 3 |
| 2 | A | 61 | 7 | | 32 | | 3 |
| 3 | A | 60 | | 2 | 38 | | 3 |
| 4 | A | 72 | 1 | | 27 | | 3 |
| 5 | B | 37 | 26 | | 37 | | 3 |
| 6 | A | | 8 | | 39 | 53 | 3 |
| 7 | A | | | 5 | 40 | 55 | 3 |
| 8 | A | | 15 | | 35 | 50 | 3 |

Table 1 (continued)

| Prep. Ex. | Prous base member | Properties of ion-exchange membranes | | | | | |
|---|---|---|---|---|---|---|---|
| | | Ion-exchange group | Ion-exchange capacity [mmol/g - dry membrane] | Water content [%] | Fixed ion concentration [mmol/g - water] | Membrane resistance [$\Omega \cdot cm^2$] | Membrane thickness [μm] |
| 1 | A | quaternary ammonium type | 0.60 | 14 | 4.3 | 0.50 | 32 |
| 2 | A | quaternary ammonium type | 0.86 | 28 | 3.1 | 0.19 | 32 |
| 3 | A | quaternary ammonium type | 0.77 | 19 | 4.1 | 0.14 | 30 |
| 4 | A | quaternary ammonium type | 0.95 | 20 | 4.8 | 0.15 | 28 |
| 5 | B | quaternary ammonium type | 0.85 | 72 | 1.2 | 0.35 | 150 |
| 6 | A | phosphoric acid type | 0.54 | 14 | 3.9 | 4.6 | 31 |
| 7 | A | phosphoric acid type | 0.55 | 28 | 2.0 | 3.4 | 31 |
| 8 | A | phosphoric acid type | 0.20 | 14 | 1.4 | 3.8 | 29 |

<note>

Porous base member:

A: Porous film,
polyethylene having a weight average molecular weight of 250,000,
a film thickness of 25 μm,
an average pore size of 0.03 μm,
a percentage of voids of 37%.

B: Nonwoven fabric,
polypropylene/polyethylene composite fiber,
apparent film thickness of 180 μm,
weight of 70 $g/m^2$,
percentage of voids of 65%.

DMC: N,N-dimethylaminoethyl methacrylate/methyl chloride (80% aqueous solution)
9EG: Nonaethylene glycol dimethacrylate
4EG: Tetraethylene glycol dimethacrylate
HEMA: Hydroxyethyl methacrylate
P-1M: 2-(Meth)acryloyloxyethyl dihydrogenphosphate
PO: t-Butylperoxyethyl hexanoate

(Comparative Preparation Example 1)

A polymerizable monomer composition (polymerizable solution) was prepared by mixing the components according to the following recipe.

Chloromethylstyrene: 380 g
Divinylbenzene: 20 g
t-Butylperoxyethyl hexanoate: 20 g

420 Grams of the above polymerizable monomer composition was introduced into a 500-ml glass container, and in which a porous drawn film (same as the one used in Preparation Example 1) measuring 20 cm x 20 cm was immersed under the atmospheric pressure at 25°C for 10 minutes, so that the porous drawn film has imbibed the monomer composition and that the voids were filled with the monomer composition.

Next, the porous drawn film was taken out from the monomer composition, and both sides of the film were covered with a polyester film of 100 μm, followed by the thermal polymerization under a nitrogen pressure of 3 $kg/cm^2$ at 80°C for 5 hours to obtain a membrane. Thereafter, the obtained membrane was reacted in an aminating bath comprising 10 parts by weight of a 30 mass% trimethylamine, 5 parts by weight of water and 5 parts by weight of acetone, at room temperature for 5 hours to obtain a quaternary ammonium-type anion-exchange membrane.

(Comparative Preparation Example 2)

A monomer composition shown in Table 2 was imbibed by the porous drawn film in the same manner as in Comparative Preparation Example 1. Next, the film was taken out from the monomer composition, and both sides of the porous film were covered with a polyester film of 100 μm, followed by the thermal polymerization under a nitrogen pressure of 3 $kg/cm^2$ at 80°C for 5 hours. Thereafter, the obtained membrane was immersed in a mixture of 98% concentration sulfuric acid and chlorosulfonic acid of a purity of not lower than 90% at a ratio of 1:1 at 40°C for 45 minutes to obtain a sulfonic acid-type cation-exchange membrane.

Ion-exchange membranes obtained in Comparative Preparation Example 1 and Comparative Preparation Example 2 above were measured for their properties in the same manner as in Preparation Example to obtain the results as shown in Table 2 which also shows properties of Neosepta AMX (manufactured by Tokuyama Corp.), Neosepta CMX (manufactured by Tokuyama Corp.) and Nafion NR-111 (manufactured by Dupont Co.) which are the ion-exchange membranes placed in the market. Here, Neosepta AMX and Neosepta CMX are ion-exchange membranes of a crosslinked polystyrene, while Nafion NR-111 is an ion-exchange membrane of the type of noncrosslinked fluorine-contained resin.

Table 2

| Comp. Prep. Ex. | Porous base member | Composition (weight ratio) | | | | Properties of ion-exchange membranes | | | | | |
|---|---|---|---|---|---|---|---|---|---|---|---|
| | | CMS | St | DVB | PO | Ion-exchange group | Ion-exchange capacity [mmol/g -dry membrane] | Water content [%] | Fixed ion concentration [mmol/g -water] | Membrane resistance [$\Omega \cdot cm^2$] | Membrane thickness [$\mu$m] |
| 1 | A | 95 | 0 | 5 | 5 | quaternary ammonium type | 1.8 | 22 | 8.2 | 0.08 | 32 |
| 2 | A | 0 | 90 | 10 | 5 | sulfonic acid type | 2.4 | 29 | 8.3 | 0.08 | 31 |
| Neosepta AMX | woven fabric | | | | | quaternary ammonium type | 1.5 | 25 | 6.0 | 0.35 | 150 |
| Neosepta CMX | woven fabric | | | | | sulfonic acid type | 1.6 | 28 | 5.7 | 0.36 | 160 |
| Nafion NR-111 | none | | | | | sulfonic acid type | 0.9 | 5 | 18.0 | 0.05 | 25 |

<note>
Porous film:
A: Porous film, polyethylene having a weight average molecular weight of 250,000, a film thickness of 25 $\mu$m, an average pore size of 0.03 $\mu$m, a percentage of voids of 37%.
CMS: Chloromethylstyrene.
St: Styrene
DVB: Divinylbenzene
PO: t-Butylperoxyethyl hexanoate

(Examples 1 to 5)

The amounts of permeation of the medicine were measured by using a virtual skin under the conditions of using ion-exchange membranes (membranes to be measured) prepared in Preparation Examples 1 to 5, filling a medicinal solution chamber of the testing apparatus of Fig. 2 with a 10 mmol/l solution of a dexamethasone phosphate disodium salt as an anionic medicine, and flowing a current of a density of 0.5 $mA/cm^2$ constant. The results were as shown in Table 3.

(Comparative Example 1)

The amount of permeation of the medicine was measured in the same manner as in Example 1 but using the Neosepta AMX (manufactured by Tokuyama Co., membrane properties are as described in Table 1) which was an anion-exchange membrane as the ion-exchange membrane using, as the base member, the woven fabric used in the conventional iontophoresis. The results were as shown in Table 3.

(Comparative Example 2)

The amount of permeation of the medicine was measured in the same manner as in Example 1 but using the anion-exchange membrane prepared in Comparative Preparation Example 1 as the ion-exchange membrane having a styrene-type ion-exchange resin. The results were as shown in Table 3.

(Comparative Example 3)

The amount of permeation of the medicine was measured in the same manner as in Example 1 by using the virtual skin only but without using the ion-exchange membrane to be measured (without using the membrane to be tested). The results were as shown in Table 3.

<Table 3>

| | Ion-exchange membranes to be measured | Medicine concentration [mmol/l] | Current density [$mA/cm^2$] | Amount of permeation [$\mu mol/cm^2$] |
|---|---|---|---|---|
| Example 1 | Preparation Example 1 | 10 | 0.5 | 2.0 |
| Example 2 | Preparation Example 2 | 10 | 0.5 | 2.8 |
| Example 3 | Preparation Example 3 | 10 | 0.5 | 3.4 |
| Example 4 | Preparation Example 4 | 10 | 0.5 | 3.1 |
| Example 5 | Preparation Example 5 | 10 | 0.5 | 2.5 |
| Comparative Example 1 | Neosepta AMX | 10 | 0.5 | 0 |
| Comparative Example 2 | Comparative Preparation Example 1 | 10 | 0.5 | 0.02 |
| Comparative Example 3 | none | 10 | 0.5 | 1.5 |
| Medicine: Dexamethasone phosphate sodium salt | | | | |

(Example 6, Comparative Examples 4 and 5)

Amounts of permeation of the medicine were measured by using the living body skin under the conditions of

using ion-exchange membranes (membranes to be measired) shown in Fig. 4, a 10 mmol/l solution of a dexamethasone phosphate disodium salt as an anionic medicine and flowing a current of a density of 0.5 mA/$cm^2$ constant. As living body skin, there was used a skin of a back portion of a micropig (Yucatane micropig, five months old, female). The results were as shown in Table 4.

<Table 4>

| | Ion-exchange membranes to be measured | Medicine concentration [mmol/l] | Current density [mA/$cm^2$] | Amount of permeation [μmol/$cm^2$] |
|---|---|---|---|---|
| Example 6 | Preparation Example 4 | 10 | 0.5 | 4.2 |
| Comparative Example 5 | Neosepta AMX | 10 | 0.5 | 0 |
| Comparative Example 6 | Comparative Preparation Example 1 | 10 | 0.5 | 0.05 |
| Medicine: Dexamethasone phosphate sodium salt | | | | |

(Examples 7 to 9, Comparative Examples 6 to 9)

Amounts of permeation of the medicine were measured by using the ion-exchange membranes (membranes to be measured) shown in Table 5, a 10 mmol/l solution of a lidocaine hydrochloride as a cationic medicine and flowing a current at a current density of 0.5 mA/$cm^2$ constant. The results were as shown in Table 5.

<Table 5>

| | Ion-exchange membranes to be measured | Medicine concentration [mmol/l] | Current density [mA/$cm^2$] | Amount of permeation [μmol/$cm^2$] |
|---|---|---|---|---|
| Example 7 | Preparation Example 6 | 10 | 0.5 | 30 |
| Example 8 | Preparation Example 7 | 10 | 0.5 | 31 |
| Example 9 | Preparation Example 8 | 10 | 0.5 | 31 |
| Comparative Example 6 | Neosepta AMX | 10 | 0.5 | 17 |
| Comparative Example 7 | Comparative Preparation Example 2 | 10 | 0.5 | 20 |
| Comparative Example 8 | None | 10 | 0.5 | 1.3 |
| Comparative Example 9 | NaFion NR-111 | 10 | 0.5 | 11 |
| Medicine: Lidocaine hydrochlorid | | | | |

## Claims

1. An iontophoresis device comprising (A) a working electrode structure being equipped with a working electrode, an ion-exchange membrane and a medicine-containing portion which contains an ionic medicine, (B) a counter electrode structure being equipped with an electrode opposing said working electrode and (C) a power source unit electrically

connected to the working electrode structure and to the counter electrode structure, said ionic medicine being permeated into a living body by electrophoresis through the ion-exchange membrane;
wherein said ion-exchange membrane has, as an ion-exchange resin, a crosslinked (meth)acrylic resin having a (meth)acrylic structural unit A to which an ion-exchange group is bonded.

**2.** The iontophoresis device according to claim 1, wherein said (meth)acrylic structural unit A is represented by the following formula (1):

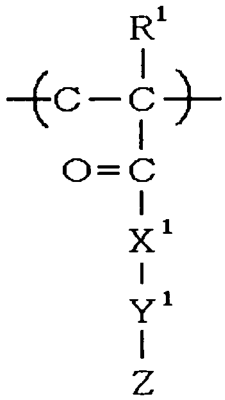
(1)

wherein,

$R^1$ is a hydrogen atom or a methyl group,
$X^1$ is -O- or >NR' (where R' is a hydrogen atom or a monovalent organic group without ion-exchange group),
$Y^1$ is a bonding hand or a divalent organic group, and
Z is an ion-exchange group.

**3.** The iontophoresis device according to claim 2, wherein said crosslinked (meth)acrylic resin further has a structural unit B represented by the following formula (2) and a structural unit C represented by the following formula (3):

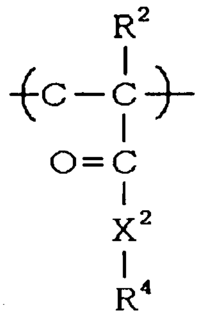
(2)

wherein,

$R^2$ is a hydrogen atom or a methyl group,
$x^2$ is -O- or >NR' (where R' is as defined above), and
$R^4$ is a monovalent organic group without ion-exchange group,

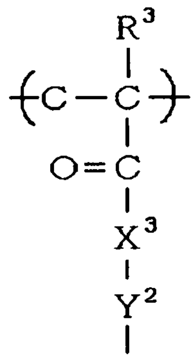

(3)

wherein,

$R^3$ is a hydrogen atom or a methyl group,
$X^3$ is - O - or NR' (where R' is as defined above), and
$Y^2$ is a divalent organic group forming a crosslinked chain.

**4.** The iontophoresis device according to claim 3, wherein when the sum of these structural units A to C is regarded to be 1, said structural unit A is contained at a ratio of 0.05 to 0.9995, said structural unit B is contained at a ratio of 0 to 0.9495, and said structural unit C is contained at a ratio of 0.0005 to 0.95.

**5.** The iontophoresis device according to claim 1, wherein said ion-exchange resin of said ion-exchange membrane is filled in voids of the porous base member.

**6.** An ion-exchange membrane for iontophoresis having, as an ion-exchange resin, a crosslinked (meth)acrylic resin that has a (meth)acrylic structural unit A to which an ion-exchange group is bonded.

**7.** The ion-exchange membrane for iontophoresis according to claim 6, wherein said ion-exchange resin is filled in voids of a porous base member.

**8.** A method of producing an ion-exchange membrane for iontophoresis comprising steps of:

contacting to a porous base member, a polymerizable solution that contains a crosslinking agent, a polymerization initiator and a polymerizable monomer composition containing a (meth)acrylic acid derivative that has an ion-exchange group, so as to permeate the polymerizable solution into voids in the porous member; and polymerizing the polymerizable solution.

Fig. 1

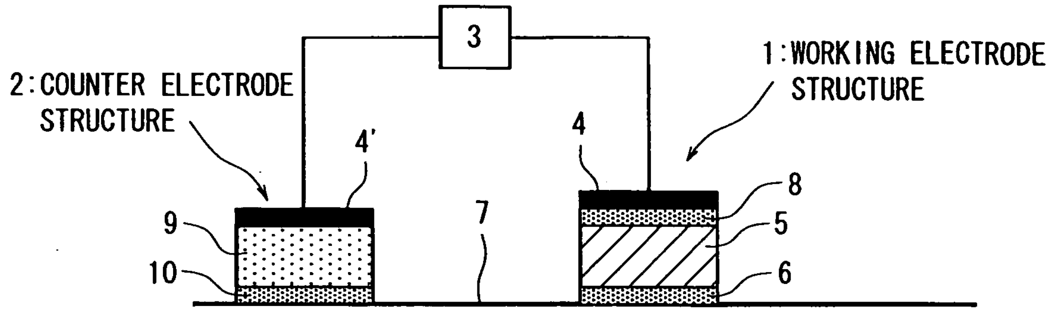
3
2:COUNTER ELECTRODE STRUCTURE
1:WORKING ELECTRODE STRUCTURE
4'
4
8
9
7
5
10
6

Fig. 2

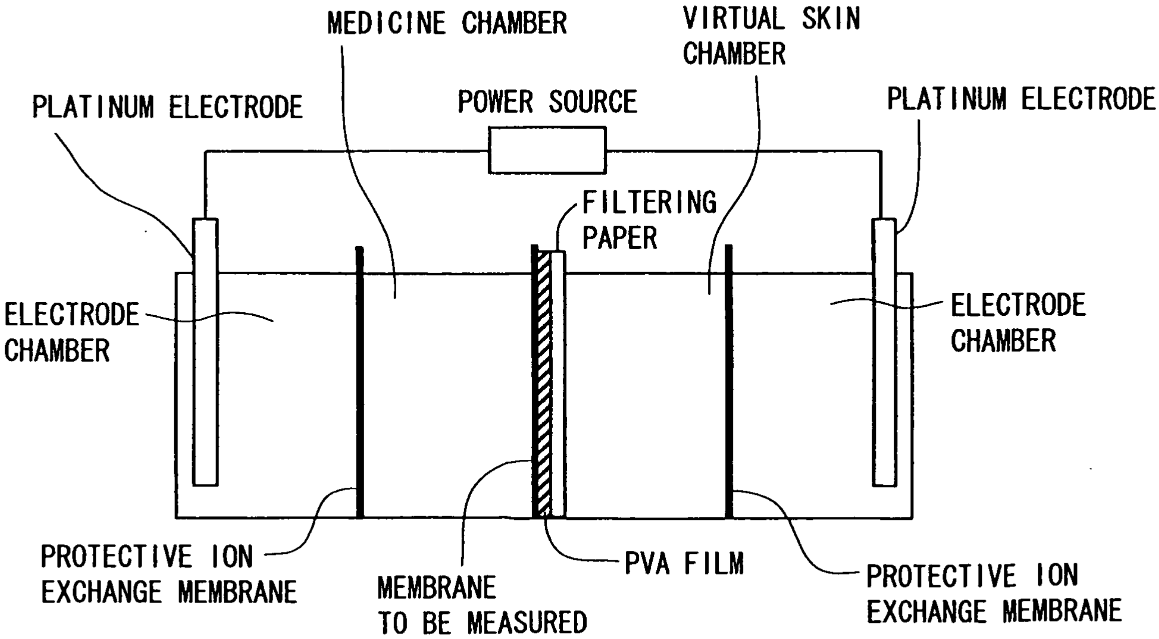
MEDICINE CHAMBER
VIRTUAL SKIN CHAMBER
PLATINUM ELECTRODE
POWER SOURCE
PLATINUM ELECTRODE
FILTERING PAPER
ELECTRODE CHAMBER
ELECTRODE CHAMBER
PROTECTIVE ION EXCHANGE MEMBRANE
MEMBRANE TO BE MEASURED
PVA FILM
PROTECTIVE ION EXCHANGE MEMBRANE

Fig. 3

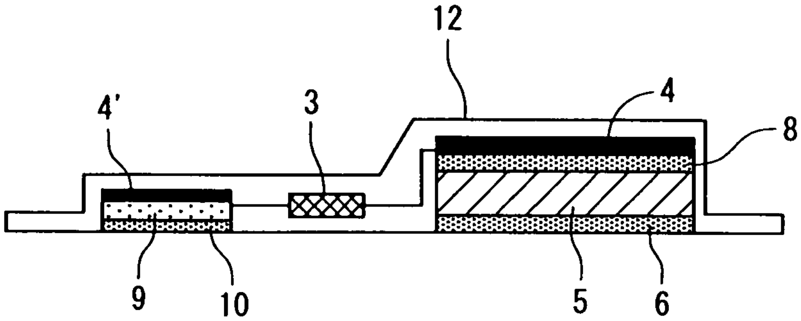

## INTERNATIONAL SEARCH REPORT

International application No.
PCT/JP2005/010009

A. CLASSIFICATION OF SUBJECT MATTER
Int.Cl[7] A61N1/30, C08J5/22

According to International Patent Classification (IPC) or to both national classification and IPC

B. FIELDS SEARCHED

Minimum documentation searched (classification system followed by classification symbols)
Int.Cl[7] A61N1/30, C08J5/22

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2000-229128 A (R and R Ventures Kabushiki Kaisha), 22 August, 2000 (22.08.00), Claim 1 (Family: none) | 1-3,5-8 |
| Y | WO 2002/093676 A2 (BALLARD POWER SYSTEMS INC.), 21 November, 2002 (21.11.02), Page 1, line 4; page 15, lines 3 to 4 & JP 2004-529472 A | 1-3,5-8 |
| Y | JP 8-245801 A (Nippon Kasei Chemical Co., Ltd.), 24 September, 1996 (24.09.96), Claim 2; Par. No. [0033] (Family: none) | 1-3,5-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 August, 2005 (02.08.05) | 23 August, 2005 (23.08.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2005/010009

C (Continuation). DOCUMENTS CONSIDERED TO BE RELEVANT

| Category* | Citation of document, with indication, where appropriate, of the relevant passages | Relevant to claim No. |
|---|---|---|
| Y | JP 62-159639 A (Sekisui Chemical Co., Ltd.), 15 July, 1987 (15.07.87), Claim 8 (Family: none) | 1-3,5-8 |
| Y | JP 2002-536073 A (FIRST WATER LTD.), 29 October, 2002 (29.10.02), Claim 12; Par. Nos. [0062], [0109] & WO 2000/045864 A1 | 1-3,5-8 |

Form PCT/ISA/210 (continuation of second sheet) (January 2004)

INTERNATIONAL SEARCH REPORT

International application No.
PCT/JP2005/010009

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 4
because they relate to parts of the international application that do not comply with the prescribed requirements to such an extent that no meaningful international search can be carried out, specifically:
The expression "the above structural unit (B) of 0 to 0.9495" of claim 4, as those wherein the structural unit (B) is 0 are included in the object of rights, is contradictory to the language to the effect that the structural (continued to extra sheet)

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest.

☐ No protest accompanied the payment of additional search fees.

Form PCT/ISA/210 (continuation of first sheet (2)) (January 2004)

INTERNATIONAL SEARCH REPORT

International application No.
PCT/JP2005/010009

Continuation of Box No.II-2 of continuation of first sheet(2)

unit (B) of claim 3 quoted by claim 4 is had. Further, in the expression "the above structural unit (B) of 0.0005 to 0.95" of claim 4, it is unclear which structural unit is referred to by "the above structural unit", so that the object of rights cannot be identified. Therefore, the requirement of clarity pursuant to PCT Article 6 is not satisfied.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3094771 A
- JP 3504343 T
- JP 4297277 A
- JP 2000229128 A
- JP 9212964 A
- JP 2002338721 A